# EUROPEAN PATENT APPLICATION

(11) **EP 4 053 281 A1**
(43) Date of publication of application: **07.09.2022**
(21) Application number: 20880789.1
(22) Date of filing: 26.06.2020
(51) Int. Cl.: C12N 15/70, C12N 9/80, C12P 13/22

(54) **STRAIN HAVING IMPROVED AMINO ACID PRODUCTION CAPABILITIES DUE TO GLSB GENE INACTIVATION, AND PRODUCTION METHOD FOR SAME**

(30) Priority: 31.10.2019 KR 20190138234
(71) Applicant: Daesang Corporation, Seoul 02586 (KR)
(72) Inventor: SHIN, Won Joo, Icheon-si Gyeonggi-do 17384 (KR); JO, Young Il, Seoul 04995 (KR); LEE, Sun Hee, Yongin-si Gyeonggi-do 16923 (KR); KIM, Hyun Young, Yongin-si Gyeonggi-do 17095 (KR); KIM, Yong Soo, Suwon-si Gyeonggi-do 16694 (KR); YANG, Cheol Min, Seoul 07958 (KR)
(74) Representative: Nony
(86) International application number: PCT/KR2020/008407
(87) International publication number: WO 2021/085796

(57) **Abstract**

Provided is a mutant strain having improved aromatic amino acid production capability as a result of inactivation or weakening of activity of glutaminase which is expressed by glutaminase B (glsB) gene.

<110>     Daesang Corporation

<120>     Strain with Improved Aromatic Amino Acid Production Capacity by
          glsB Gene Inactivation

<130>     PX200025PCT

<150>     KR 10-2019-0138234
<151>     2019-10-31

<160>     9

<170>     KoPatentIn 3.0

<210>     1
<211>     927
<212>     DNA
<213>     Artificial Sequence

<220>
<223>     glsB "G6810-MONOMER" (complement(1612325..1613251)) Escherichia
          coli K-12 substr. MG1655

<400>     1
gtggcagtcg ccatggataa tgcaatttta gaaaacatct tgcggcaagt gcggccgctc          60

attggtcagg gtaaagtcgc ggattatatt ccggcgctgg ctacagtaga cggttcccga         120

ttggggattg ctatctgtac cgttgacgga cagctttttc aggccggaga cgcgcaagaa          180

cgtttttcca ttcagtctat ttccaaagtg ctgagtctcg ttgtcgccat gcgtcattac         240

tccgaagagg aaatctggca acgcgtcggc aaagatccgt ctggatcacc gttcaattcc         300

ttagtgcaac tggaaatgga gcagggtata ccgcgtaatc cgttcattaa tgccggtgcg         360

ctggtggtct gcgatatgtt gcaagggcga ttaagcgcac cacggcaacg tatgctggaa         420

gtcgtgcgcg gcttaagcgg tgtgtctgat atttcctacg atacggtggt agcgcgttcc         480

gaatttgaac attccgcgcg aaatgcggct atcgcctggc tgatgaagtc gtttggcaat         540

ttccatcatg acgtgacaac cgttctgcaa aactactttc attactgcgc tctgaaaatg         600

agctgtgtag agctggcccg gacgtttgtc tttctggcta atcaggggaa agctattcat         660

attgatgaac cagtggtgac gccaatgcag gcgcggcaaa ttaacgcgct gatggcgacc         720

agtggtatgt accagaacgc gggggagttt gcctggcggg tggggctacc ggcgaaatct         780

ggcgttggtg gcggtattgt ggcgattgtt ccgcatgaaa tggccatcgc tgtctggagt         840

ccggaactgg atgatgcagg taactcgctt gcgggtattg ccgttcttga acaattgacg         900

aaacagttag ggcgttcggt ttattaa                                             927

<210>     2
<211>     20
<212>     DNA
<213>     Artificial Sequence

<220>
<223>    glsB_HF1


<400>    2
gatagtgagt tcggcctttc                                                    20


<210>    3
<211>    20
<212>    DNA
<213>    Artificial Sequence

<220>
<223>    glsB_HR1


<400>    3
tttctactcc tggaccgcag                                                    20


<210>    4
<211>    40
<212>    DNA
<213>    Artificial Sequence

<220>
<223>    glsB_PF


<400>    4
ctgcggtcca ggagtagaaa gtgtaggctg gagctgcttc                              40


<210>    5
<211>    40
<212>    DNA
<213>    Artificial Sequence

<220>
<223>    glsB_PR


<400>    5
agtggatcga gagactgcat ctgtcaaaca tgagaattaa                              40


<210>    6
<211>    20
<212>    DNA
<213>    Artificial Sequence

<220>
<223>    glsB_HF2


<400>    6
atgcagtctc tcgatccact                                                    20


<210>    7
<211>    20
<212>    DNA

<213>    Artificial Sequence

<220>
<223>    glsB_HR2


<400>    7
accgccacga tataacgttg                                                    20


<210>    8
<211>    20
<212>    DNA
<213>    Artificial Sequence

<220>
<223>    glsB_CF


<400>    8
atcaggtgga gaaaaccctg                                                    20


<210>    9
<211>    20
<212>    DNA
<213>    Artificial Sequence

<220>
<223>    glsB_CR


<400>    9
tgaaccagtc cgcaagcaaa                                                    20

## Description

### Technical Field

The present invention relates to a strain having improved aromatic amino acid production capability due to inactivation of glsB gene.

### Background Art

Aromatic amino acids, particularly L-tryptophan and L-phenylalanine, are important amino acids for feed and are high value-added industries having an annual worldwide market size of 300 billion dollars.

Aromatic amino acids are produced using recombinant strains, and studies have been actively conducted to increase their production. Chorismate is a precursor required in the aromatic amino acid biosynthesis pathways, and in order to produce chorismate, phosphoenolpyruvate (PEP), erythrose-4-phosphate (E4P), the sub-substrate phosphoribosyl pyrophosphate (PRPP), serine, glutamine and the like are needed. Thus, in a conventional art, studies on enhancement of the biosynthetic pathway of E4P, PEP or PRPP have been conducted to improve L-tryptophan production.

However, it has not been reported that the production of aromatic amino acids by a strain is increased by inhibiting the expression of glutaminase B (glsB) gene in the strain.

### [Prior Art Documents]

(Patent Document 0001) Korean Patent No. 10-1830002 (February 9, 2018)

### DISCLOSURE

### Technical Problem

According to one embodiment, there is provided a strain having improved aromatic amino acid production capability as a result of inhibiting the expression of the glsB gene.

### Technical Solution

One aspect provides a mutant strain having improved aromatic amino acid production capability by inactivation or weakening of activity of glutaminase which is expressed by glutaminase B (glsB) gene.

The glsB gene may consist of the nucleotide sequence of SEQ ID NO: 1.

The relationship of the glsB gene with an aromatic amino acid production pathway such as the tryptophan pathway, or the effect of the glsB gene on the aromatic amino acid production pathway is unknown. Nevertheless, the present inventors have found that, when the activity of glutaminase in a strain is inhibited by inhibiting the expression of the glsB gene in the strain, energy that is used for glutamate production may decrease, and the aromatic amino acid productivity of the strain may be improved.

As used herein, the term "weakening of activity" means that the expression level of a gene of interest is decreased compared to the original expression level thereof. This weakening of activity includes: a case in which the activity of an enzyme itself is decreased compared to the activity of the enzyme, originally possessed by the microorganism, through substitution, insertion or deletion of one or more nucleotides in the nucleotide sequence of the gene encoding the enzyme, or a combination thereof; a case in which the overall activity of the enzyme in the cell is lower than that in a native strain or a strain before modification due to inhibition of expression or translation of the gene encoding the enzyme; and a combination thereof.

As used herein, the term "inactivation" refers to a case in which a gene encoding a protein such as an enzyme is not expressed at all compared to that in a native strain or a strain before modification, and has no activity even if it is expressed.

As used herein, the term "increased expression" means that the expression level of a gene of interest is increased compared to the original expression level of the gene. If a gene whose expression is to be increased is not present in a strain before mutation, the expression may be increased by introducing one or more genes into the chromosome of the strain, and if a gene whose expression is to be increased is present in a strain before mutation, one or more genes may be additionally introduced into the strain, or the strain may be genetically engineered to increase the expression level of the existing gene.

In the present invention, a method of modifying an expression regulatory sequence may be performed by inducing a modification in the expression regulatory sequence by deletion, insertion, or non-conservative or conservative substitution of one or more nucleotides in the nucleic acid sequence of the expression regulatory sequence, or a combination thereof, or may be performed by substituting the sequence with a weaker promoter. Examples of the expression regulatory sequence include a promoter, an operator sequence, a sequence encoding a ribosome-binding site, and a sequence for regulating the termination of transcription and translation.

In addition, a method of modifying a gene sequence on the chromosome may be performed by inducing a modification in the sequence by deletion, insertion, non-conservative or conservative substitution, or a combination thereof in the gene sequence so as to further weaken the enzyme activity, or may be performed by replacing the sequence with a gene sequence improved to have weaker activity or with a gene sequence improved to have no activity.

According to one embodiment, the aromatic amino acid may be at least one of L-tyrosine, L-tryptophan, and L-phenylalanine.

According to one embodiment, the mutant strain may be obtained by insertion, substitution or deletion of one or more nucleotides in the nucleotide sequence of the glsB gene.

According to one embodiment, the mutant strain may be derived from a strain of the genus Escherichia.

According to one embodiment, the strain of the genus Escherichia may be *Escherichia coli,* for example, a strain deposited under accession number KFCC11660P or KCCM10016.

Another aspect provides a method for producing an aromatic amino acid, comprising steps of: culturing the mutant strain in a medium; and recovering an aromatic amino acid from the cultured strain and the medium.

The strain according to the present invention may be cultured through a culture method known in the art. As the medium, a natural medium or a synthetic medium may be used. Examples of the carbon source of the medium include glucose, sucrose, dextrin, glycerol, starch, etc., and examples of the nitrogen source of the medium include peptone, meat extract, yeast extract, dried yeast, soybean cake, urea, thiourea, ammonium salt, nitrate and other organic or inorganic nitrogen-containing compounds, but the carbon and nitrogen sources are not limited to these components.

As inorganic salts contained in the medium, phosphates, nitrates, carbonates, chlorides, etc. of magnesium, manganese, potassium, calcium, iron, etc. may be used, without being limited thereto. In addition to the components of the carbon source, nitrogen source and inorganic salt, amino acids, vitamins, nucleic acids and related compounds may be added to the medium.

The temperature of the culture medium may be usually 27 to 40°C, more preferably 30 to 37°C, without being limited thereto. The culture may be continued until the useful substances are produced in desired amounts. The culture time may preferably be 10 to 100 hours, without being limited thereto.

In the step of recovering the aromatic amino acid, the desired amino acid may be recovered from the culture medium using a suitable method known in the art, depending on the method used for culture of the microorganism of the present invention, for example, a batch, continuous or fed-batch culture method. The step of recovering may include a purification process.

According to one embodiment, the aromatic amino acid may be at least one of L-tryptophan and L-phenylalanine.

### Advantageous Effects

According to one embodiment, it is possible to increase the aromatic amino acid production of the strain by inactivating the glsB gene in the strain.

### Mode for Invention

Hereinafter, one or more specific embodiments will be described in more detail with reference to examples. However, these examples are for illustrating one or more embodiments, and the scope of the present invention is not limited to these examples.

### Example 1: Construction of glsB gene-deleted mutant strains

glsB gene-inactivated mutant strains were constructed from parent strains (accession numbers: KFCC11660P and KCCM10016) by a one-step inactivation method (One-step inactivation of chromosomal genes in Escherichia coli K-12 using PCR products, Datsenko KA, Wanner BL., Proc Natl Acad Sci USA. 2000 Jun 6;97(12):6640-5).

The KFCC11660P strain and the KCCM10016 strain are *Escherichia coli* strains. For homologous recombination of the fourth fragment, pKD46 (GenBank accession number: AY048746), a Red recombinase plasmid, was introduced into each of the strains, and pKD46 was removed before introduction of pCP20.

The glsB gene was deleted by homologous recombination between the glsB gene and a DNA fragment containing an antibiotic resistance gene, and then the glsB gene was inactivated by removing the antibiotic resistance gene from the recombined DNA fragment. The specific process is as follows.

### (1) Construction of first fragment

PCR reaction (total volume: 50 µl) was performed using a pKD13 plasmid (Genbank accession number: AY048744) and a primer pair of glsB_PF and glsB_PR having a portion of the glsB gene sequence shown in Table 1 below and a portion of the pKD13 plasmid sequence under the following conditions, thus obtaining a first amplified fragment of about 1.4 kb in length: one cycle of 5 min at 95°C, and then 30 cycles, each consisting of 30 sec at 95°C, 30 sec at 58°C, and 2 min at 72°C, followed by 5 min at 72°C and 10 min at 12°C. The first fragment contained the kanamycin resistance gene derived from the pKD13 plasmid.

**[Table 1]**

| | SEQD NO | Sequence |
|---|---|---|
| glsB | 1 | gtggcagtcgccatggataatgcaattttagaaaacatcttgcggcaagtgcggccgctca ttggtcagggtaaagtcgcggattatattccggcgctggctacagtagacggttcccgattg gggattgctatctgtaccgttgacggacagctttttcaggccggagacgcgcaagaacgttt ttccattcagtctatttccaaagtgctgagtctcgttgtcgccatgcgtcattactccgaagag gaaatctggcaacgcgtcggcaaagatccgtctggatcaccgttcaattccttagtgcaact |
| | | ggaaatggagcagggtataccgcgtaatccgttcattaatgccggtgcgctggtggtctgc gatatgttgcaagggcgattaagcgcaccacggcaacgtatgctggaagtcgtgcgcgg cttaagcggtgtgtctgatatttcctacgatacggtggtagcgcgttccgaatttgaacattcc gcgcgaaatgcggctatcgcctggctgatgaagtcgtttggcaatttccatcatgacgtgac aaccgttctgcaaaactactttcattactgcgctctgaaaatgagctgtgtagagctggcccg gacgtttgtctttctggctaatcaggggaaagctattcatattgatgaaccagtggtgacgcc aatgcaggcgcggcaaattaacgcgctgatggcgaccagtggtatgtaccagaacgcgg gggagtttgcctggcgggtggggctaccggcgaaatctggcgttggtggcggtattgtgg cgattgttccgcatgaaatggccatcgctgtctggagtccggaactggatgatgcaggtaa ctcgcttgcgggtattgccgttcttgaacaattgacgaaacagttagggcgttcggtttattaa |
| glsB_HF1 | 2 | gatagtgagt tcggcctttc |
| glsB_HR1 | 3 | tttctactcc tggaccgcag |
| glsB_PF | 4 | ctgcggtcca ggagtagaaa gtgtaggctg gagctgcttc |
| glsB_PR | 5 | agtggatcga gagactgcat ctgtcaaaca tgagaattaa |
| glsB_HF2 | 6 | atgcagtctc tcgatccact |
| glsB_HR2 | 7 | accgccacga tataacgttg |
| glsB_CF | 8 | atcaggtgga gaaaaccctg |
| glsB_CR | 9 | tgaaccagtc cgcaagcaaa |

### (2) Construction of second fragment

To obtain an upstream fragment of the glsB gene, PCR reaction (total volume: 50 µl) was performed using the genomic DNA of *E. coli* MG1655 as a template and the primers glsB_HF1 and glsB_HR1 shown in Table 1 above under the following conditions, thus obtaining a second amplified fragment of about 0.3 kb in length: one cycle of 5 min at 95°C, and then 30 cycles, each consisting of 30 sec at 95°C, 30 sec at 58°C, and 30 sec at 72°C, followed by 5 min at 72°C and 10 min at 12°C.

### (3) Construction of third fragment

To obtain a downstream fragment of the glsB gene, PCR reaction (total volume: 50 µl) was performed using the genomic DNA of *E. coli* MG1655 as a template and the primers glsB_HF2 and glsB_HR2 shown in Table 1 above under the following conditions, thus obtaining a third amplified fragment of about 0.3 kb in length: one cycle of 5 min at 95°C, and then 30 cycles, each consisting of 30 sec at 95°C, 30 sec at 58°C, and 30 sec at 72°C, followed by min at 72°C and 10 min at 12°C.

### (4) Construction of fourth fragment

The first fragment, second fragment and third fragment amplified in the above experiment could be ligated into a single fragment due to the complementary sequences of the primers during amplification. These fragments were subjected to PCR (total volume: 50 µl) without primers under the following conditions, thus obtaining a fourth amplified single fragment having a size of about 2 kb: one cycle of 5 min at 95°C, and then 30 cycles, each consisting of 30 sec at 95°C, 30 sec at 58°C, and 2 min and 30 sec at 72°C, followed by 5 min at 72°C and 10 min at 12°C. The fourth fragment contained a portion of the glsB gene and the kanamycin antibiotic resistance gene. Specifically, it consisted of a portion of the 5' fragment of the glsB gene, the kanamycin antibiotic resistance gene, and a portion of the 3' fragment of the glsB gene.

### (5) Introduction of fourth fragment and deletion of glsB

The obtained fourth fragment was introduced by electroporation into each of the KFCC11660P and KCCM10016 strains, which are *Escherichia coli* strains containing the Red recombinase plasmid pKD46 (GenBank accession number: AY048746). The fourth fragment was replaced with glsB by homologous recombination using the Lambda Red recombination system, whereby glsB was deleted.

Thereafter, PCR reaction was performed on the cell line showing kanamycin resistance to confirm whether the glsB gene was deleted. The PCR reaction (total volume: 20 µl) was performed using the glsB_CF and glsB_CR primers shown in Table 1 above under the following conditions: one cycle of 5 min at 95°C, and then 30 cycles, each consisting of 30 sec at 95°C, 30 sec at 55°C, and 3 min at 72°C, followed by 5 min at 72°C and 10 min at 12°C. It was confirmed that, when the original glsB gene was present, about 1.9 kb (before deletion) was produced, whereas when the fragment was inserted into the chromosome, about 2.3 kb (containing the antibiotic resistance gene) which is an increased length was produced.

### (6) Antibiotic resistance gene removal and selection

To remove the antibiotic resistance marker gene from the strain in which deletion of the glsB gene was confirmed, FLP recombination was induced by introducing a pCP20 plasmid into the strain. Thereafter, the glsB-deleted strain was cultured in LB plate medium with or without antibiotics to confirm that the antibiotic resistance marker gene was removed.

### Example 2: Evaluation of aromatic amino acid production of glsB-deleted strains

Each of the *E. coli* strain KFCC11660PΔglsB obtained by the method of Example 1 and KFCC11660P was cultured in the tryptophan-producing medium shown in Table 3 below.

In addition, each of the *E. coli* strain KCCM10016ΔglsB obtained by the method of Example 1 and KCCM10016 was cultured in the phenylalanine-producing medium shown in Table 2 below.

For culture, 1 vol% of each of the KFCC11660PΔglsB, KFCC11660P, KCCM10016ΔglsB, and KCCM10016 strains was inoculated into a flask containing 10 mL of the tryptophan-producing medium or phenylalanine-producing medium having the composition shown in Table 2 below, and cultured with shaking at 200 rpm at 37°C for 70 hours. Then, the concentrations of L-amino acids obtained from the strains were compared.

**[Table 2]**

| Tryptophan-producing medium | | Phenylalanine-producing medium | |
|---|---|---|---|
| Component | Content | Component | Content |
| Glucose | 80.0 g/L | Glucose | 80.0 g/L |
| (NH₄)₂SO₄ | 20.0 g/L | (NH₄)₂SO₄ | 20.0 g/L |
| K₂HPO₄ | 0.8 g/L | K₂HPO₄ | 1.0g/L |
| K₂SO₄ | 0.4 g/L | KH₂PO₄ | 1.0 g/L |
| MgCl₂ | 0.8 g/L | K₂SO₄ | 0.4 g/L |
| Fumaric acid | 1.0 g/L | MgCl₂ | 1.0 g/L |
| Yeast extract | 1.0 g/L | Fumaric acid | 0.5 g/L |
| (NH₄)₆Mo₇O₂₄ | 0.12 ppm | Yeast extract | 1.0 g/L |
| H₃BO₃ | 0.01 ppm | Glutamic acid | 0.5g/L |
| CuSO₄ | 0.01 ppm | CaCl₂ | 5.00 ppm |
| MnCl₂ | 2.00 ppm | MnCl₂ | 2.00 ppm |
| ZnSO₄ | 0.01 ppm | ZnSO₄ | 1.00 ppm |
| CoCl₂ | 0.10 ppm | CoCl₂ | 0.10 ppm |
| FeCl₂ | 10.00 ppm | FeCl₂ | 10.00 ppm |
| Thiamine_HCl | 20.00 ppm | Thiamine_HCl | 20.00 ppm |
| L-Tyrosine | 200.00 ppm | L-Tyrosine | 200.00 ppm |
| L-phenylalanine | 300.00 ppm | CaCO₃ | 3% |
| CaCO₃ | 3% | - | - |

As a result of the above experiment, as shown in Tables 3 and 4 below, it was confirmed that, in the case of the strains in which the glsB gene was inactivated, the production of tryptophan and phenylalanine increased and the production of glutamate significantly decreased.

Referring to Tables 3 and 4 below, it was confirmed that, when the glsB gene in the KFCC11660P strain was inactivated, the production of L-tryptophan increased by about 10%, and when the glsB gene in the KCCM10016 strain was inactivated, the production of L-phenylalanine increased by about 10%.

**[Table 3]**

| Strain | L-tryptophan (g/L) | L-glutamate (relative amount) |
|---|---|---|
| KFCC11660P | 4.21 | 76.39 |
| KFCC11660PΔglsB | 4.62 | 13.65 |

**[Table 4]**

| Strain | L-phenylalanine (g/L) | L-glutamate (relative amount) |
|---|---|---|
| KCCM10016 | 3.47 | 20.17 |
| KCCM10016ΔglsB | 3.81 | 8.56 |

## Claims

1. A mutant strain having improved aromatic amino acid production capability due to inactivation or weakening of activity of glutaminase which is expressed by glutaminase B (glsB) gene.

2. The mutant strain of claim 1, wherein the glsB gene consists of the nucleotide sequence of SEQ ID NO: 1.

3. The mutant strain of claim 1, wherein the aromatic amino acid is at least one of L-tryptophan and L-phenylalanine.

4. The mutant strain of claim 1, wherein the inactivation or weakening of activity of glutaminase is achieved by insertion, substitution or deletion of one or more nucleotides in the nucleotide sequence of the glsB gene.

5. The mutant strain of claim 1, which is derived from a strain of the genus Escherichia.

6. The mutant strain of claim 5, wherein the strain of the genus Escherichia is *Escherichia coli.*

7. A method for producing an aromatic amino acid, comprising steps of:
culturing the mutant strain of claim 1 in a medium; and
recovering an aromatic amino acid from the cultured mutant strain and the medium.

8. The method of claim 7, wherein the aromatic amino acid is at least one of L-tryptophan and L-phenylalanine.
